# EUROPEAN PATENT APPLICATION

(11) **EP 4 512 243 A1**
(43) Date of publication of application: **26.02.2025**
(21) Application number: 23192521.5
(22) Date of filing: 21.08.2023
(51) Int. Cl.: A01K 67/0275, C07K 16/00, C12N 15/85, C12N 15/11

(54) **BINDING MOLECULES**

(71) Applicant: Kymab Limited, Cambridge CB22 3AT (GB)
(72) Inventor: The designation of the inventor has not yet been filed
(74) Representative: CMS Cameron McKenna Nabarro Olswang LLP

(57) **Abstract**

The present disclosure provides transgenic non-human animal cells that are useful for the production of human heavy chain-only antibodies (HCAbs). Methods for producing human HCAbs specific to a desired antigen and methods for producing pharmaceutical compositions comprising an antibody specific to a desired antigen are also disclosed.

## Description

### FIELD

The disclosure relates to the production of fully human heavy chain only antibodies in non-human animal cells.

### BACKGROUND

The antigen-binding region of a native human immunoglobulin is composed of two variable domains - the heavy chain variable (VH) domain and the light chain variable (VL) domain - which pair together to form an Fv region. The Fv region has an antigen-binding site provided by six loops of variable amino acid sequence - the complementarity determining regions (CDRs). The VH domain comprises HCDR1, HCDR2 and HCDR3 interspersed with framework regions (FRs), and the VL domain comprises LCDR1, LCDR2 and LCDR3 interspersed with FRs. One or more CDRs of the VH domain and/or of the VL domain bind to the antigen. HCDR3 of the VH domain often has a major role in antigen-binding, although other CDRs of both domains can and often do contribute. Even where an antigen binds solely or mainly to CDRs of the VH domain, the presence of the VL domain in the Fv may stabilise the VH in a functional binding conformation.

Antibody variable domains are generated *in vivo* through combinatorial rearrangement of gene segments at the immunoglobulin (Ig) loci within cells of B lymphocyte lineage, which provides a repertoire of encoded amino acid sequences capable of binding to the diverse immunogenic stimuli encountered by the immune system. The Ig heavy chain locus in humans has approximately 41 functional V gene segments, 27 functional D gene segments and 6 functional J gene segments, depending on haplotype. Nucleic acid encoding a VH domain is generated through V-D-J gene segment recombination. The V gene segment encodes the N terminal region of the polypeptide chain comprising FR1, HCDR1, FR2, HCDR2, FR3 and the start of HCDR3, while the D gene segment is encompassed within HCDR3 and the J gene segment provides the end of HCDR3 and the C terminal framework region FR4. The highly variable nature of HCDR3 sequences in an antibody repertoire reflects the combinatorial diversity generated by rearrangement of the many different V, D and J segments.

Humans have two Ig light chain loci, kappa (k) and lambda (λ). The human Ig light chain loci have approximately 40 functional Vk segments, 5 functional Jk segments, 29 functional Vλ segments and 4 functional Jλ segments, depending on haplotype. Nucleic acid encoding a VL domain is produced by the recombination of two gene segments, V and J, at either the kappa (k) or lambda (λ) locus. The V gene segment encodes the FR1, LCDR1, FR2, LCDR2, FR3 and the first part of LCDR3, while the J gene segment forms the second part of LCDR3 and the FR4. In addition to the combinatorial diversity arising from V-D-J and V-J recombination and from VH/VL pairing, further antibody sequence diversity is generated by junctional mutations at the point of gene segment joining and by the *in vivo* process of somatic hypermutation in response to antigen binding.

WO90/05144 (MRC) disclosed that VH domains, when isolated from complete antibodies comprising heavy and light chains, were able to bind to antigen in a 1:1 ratio and with binding constants of equivalent magnitude to those of complete antibody molecules.

Camelids (the animal family including camels and llamas) naturally produce antibodies which bind antigen with unpaired VH domains in the absence of a VL. These antibodies completely lack the immunoglobulin light chain, and are bivalent binders composed of homodimeric heavy chains each comprising an antigen-binding single VH or "VHH" domain, a hinge region and a dimerising constant region comprising CH2 and CH3 domains. While homologous to the heavy chains of classical mammalian antibodies, these "heavy chain antibodies" ("HCAbs") lack the first domain of the constant region (CH1), which is spliced out during mRNA processing (WO94/ 04678 Casterman & Hamers; WO96/34103 Hamers & Muyldermans).

An analogous mutation occurs in a pathological setting in humans, known as heavy chain disease. Immunoglobulin heavy chains are expressed comprising the VH, CH2 and CH3 domains, but lacking a CH1 domain. In patients with this disease the heavy chains are found to accumulate instead of pairing with light chains to form normal antibodies.

Cartilaginous fish such as sharks also have antibodies composed of heavy chains and lacking any light chain. These antibodies are termed IgNAR (immunoglobulin new antigen receptor) and have been used as a source of single domain antibodies called VNAR fragments.

Researchers have worked to develop single variable domain antibodies into pharmaceutical products. These molecules, known as domain antibodies (dAbs), are bioactive as monomers and, owing to their small size and inherent stability, are also well suited for incorporation into larger molecules to create drugs with prolonged serum half-lives and/or other pharmacological improved activities. Antigen-binding molecules comprising single variable domains of antibodies include immunoconjugates (e.g., dAb-toxin) and chimeric antigen receptors (CARs). To this end, antibody single variable domain binders have been cloned and expressed in recombinant systems, and in vitro libraries of such binders have been generated for selection and screening using systems such as phage display.

Laboratory animals such as mice have been genetically engineered to express heavy chain antibodies as a further source of single antigen-binding variable domains. One can begin by knocking out (deleting or inactivating) the endogenous Ig light chain loci of the animal (WO92/03918 Genpharm; WO03/000737 The Babraham Institute). The heavy chain alone is then expressed, and homodimerises. However, the CH1 domain of the heavy chain is intrinsically disordered and adopts the typical immunoglobulin fold only upon interaction with its cognate partner, the Ck or Cλ domain of the light chain. Expression of Ig heavy chains has been reported to be non-productive owing to misfolding and aggregation of the CH1 domain.

Nevertheless, in light chain-deficient mice, some functional HCAbs are spontaneously produced through a variation of the class switch mechanism. In class switching, nucleic acid encoding the VH is normally joined to nucleic acid encoding a CH1-CH2-CH3 constant region, but in the aberrant mechanism, the VH is joined to only CH2-CH3. The resulting HCAbs, like those of camelids, lack the CH1 domain and can be expressed and selected for antigen binding. However, functional HCAbs are generated only at low efficiency by this method, since the normal mechanism of class switching predominates and produces a full Ig heavy chain including the CH1.

Mouse strains used for producing heavy chain antibodies have therefore been engineered to have a genetic deletion of the CH1 domain of the immunoglobulin heavy chain and knockout of the light chains. Such mice produce antibodies comprising dimeric heavy chains and lacking light chains, each heavy chain having a variable (VH) domain and constant regions CH2 and CH3. The constant regions dimerise to form an Fc region, while the two VH domains are available for divalent antigen binding. Following immunisation of the mice with a target antigen, heavy chain antibodies specific for the antigen are generated, selected and affinity matured *in vivo,* and can be isolated. Nucleic acid encoding the VH domains can then be expressed recombinantly and cloned as desired to provide polypeptides comprising the VH domain, optionally in the context of larger molecules such as CARs or other products for therapeutic or diagnostic use.

Erasmus Universiteit Rotterdam have described transgenic mice whose genomes comprise exons from camelid VHH domains or "camelised" VH domains and heavy chain constant region genes which did not express a functional CH1 domain (WO02/085944, WO02/085945, WO2006/008548, WO2010/109165). "Camelised" VH are human (or other non-camelid) VH sequences which have been mutated to resemble camelid VHH. In these transgenic mice, the heavy chain genes are engineered to exclude functional CH1 domains. As reported in WO02/085944, the absence of the CH1 rendered the heavy chain antibodies unable to associate with light chains to form "conventional" antibodies, since the CH1 was the natural partner for the constant domain of the light chain. WO2006/008548 reported that normal B-cell maturation and antibody production in the mice was dependent on the complete absence of CH1 sequences from each heavy chain constant region present in the transgenic locus. WO2004/049794 (The Babraham Institute) describes production of HCAbs from a YAC transgene in mice, in which the CH1 domain of the heavy chain is spliced out during mRNA processing. The resulting mice could then be crossed with mice in which endogenous heavy and light chain genes had been knocked out, to generate mice which only expressed the desired HCAbs.

Unfortunately, heavy chain-only mice carrying CH1 deletions in their Ig heavy chains do not have normal B cell populations. Mice in which DNA encoding the CH1 domain was deleted from both the µ and the γ heavy chain constant region genes produced IgM heavy chains lacking CH1 (CH1Δµ) and IgG heavy chains lacking CH1 (CH1Δy), but the proportion of immature B cells in these mice was increased, with impaired differentiation into follicular zone B cells and marginal zone B cells. (Janssens et al., PNAS (2006) 103(41):15130-15135).

Human VH domains are desirable for administration to humans for a variety of reasons. Human VH domains exhibit improved stability and are of a smaller size compared to a full-length antibody, allowing generation of antibodies directed against wider range of targets, including epitopes that conventional antibodies fail to target. Human VH domains are also associated with lower immunogenic side effects in patients compared with administration of polypeptides of non-human origin such as camelid VHH. Transgenic mice expressing human antibody heavy chains are a source of human VH domains that undergo *in vivo* selection for antigen-binding. However, human antibodies naturally contain both heavy and light chains, and only a subset of heavy chain variable regions are able to generate functional heavy chain antibodies in the absence of the light chain. Human VH domains from heavy chain antibodies produced in heavy chain only mice therefore lack the sequence diversity of VH domains from full immunoglobulins. The limitation of the human VH domain repertoire in such mice is compounded where the human Ig heavy chain genes are introduced at a random insertion point in the mouse genome, since the ectopic transgenic locus produces VH domains with HCDR3 sequences which are shorter than in humans (believed to result from limited N-addition occurring during VDJ recombination) and which undergo limited hypermutation, resulting in an already low diversity of VH domains. This appears to be an intrinsic limitation of known random insertion transgenic platforms for human antibody generation. The latter issue can be addressed by integrating the human immunoglobulin genes at the endogenous immunoglobulin locus of the host animal, rather than at a random position in the genome (Lee et al., Nature Biotech (2014) 32(43):356-363).

Recombination and somatic hypermutation at the native loci in such mice generate a broader diversity of sequences from which to evolve less soluble VH domains into ones with improved biophysical properties (such as solubility) *in vivo.*

WO2011/072204 (Regeneron Pharmaceuticals) described mice expressing heavy chain antibodies comprising human VH domains and mouse constant regions with deleted CH1 domains. These mice were said to comprise a germline deletion of the sequence encoding CH1 in an endogenous Ig constant region gene, rendering them incapable of expressing an IgG mRNA that comprised a sequence encoding a CH1 domain, but the mice retained the ability to express normal functional IgM antibody as the CH1 domain of the IgM isotype constant domain was not deleted.

WO2013/171505 (Kymab Limited) described a mouse engineered to express normal IgM antibodies and CH1-deleted IgG antibodies, where stage-specific class switching from IgM to IgG in lymphocytes was accompanied by inactivation of the endogenous Ig light chain loci and genetic deletion of CH1 from the IgG constant region, so that IgG antibodies were expressed by the cell in the absence of light chain expression. This modification enabled antibody and B-cell compartment development to pass through a favourable 4-chain (H2L2, i.e. antibody comprising two heavy chains and two light chains) endogenous IgM stage before proceeding to a subsequent IgG stage which selected solely heavy chain only (H2) antibodies from the good pool of heavy chain VDJ recombinations provided by the earlier 4-chain IgM stage, this subsequent stage essentially eliminating the possibility for 4-chain antibodies.

WO2018/039180 (Teneobio Inc.) reported that HCAbs with less propensity for aggregation could be prepared by replacement of the native amino acid residue at the first position of FR4 of an HCAb by another amino acid residue to disrupt a surface-exposed hydrophobic patch which, in a normal Fv, would be buried in the VH-VL interface. The exposure of the hydrophobic patch was identified as being a causal factor in the unwanted aggregation of heavy chains in the absence of light chain, as well as in VH-VL domain pairing in the presence of light chain. Rats were genetically engineered to express HCAbs including identified VH residue mutations, and heavy chain homodimerisation was enforced by inactivation of the endogenous light chain loci.

Xu et al (Nature (2021) 595:278-282) disclose a mouse comprising VHH genes from alpaca, dromedary and camel, comprising CH1 deletions in Cµ and Cy1, and retaining the mouse constant region, for the production of nanobodies useful for neutralising SARS-CoV-2 variants.

WO2022/126113 (Trianni Inc.) describes a mouse engineered to produce HCAbs of the IgG type by incorporating within the endogenous immunoglobulin heavy chain constant region locus a transgenic Cγ gene segment upstream of the endogenous Cµ gene segment (in 5' to 3' orientation), wherein the Cγ gene segment comprises a deletion of at least part of the CH1 domain.

EP3770261 A1 (Chongqing Jinmaibo Biotec. Co.) discloses using transgenic host animals to produce humanized single-domain antibodies. According to the disclosure, all the IgM sequences, and all IgG regulatory control sequences, are originated from the host animal and the CH1 sequence of IgM is deleted. The IgG Cγ sequences are human sequences, and the CH1 sequence of IgG is deleted. The inventors aim to ensure normal B cell development in the transgenic animal and disclose that the use of host animal genes are necessary to ensure proper B cell development.

There remains a need in the art for new approaches to generating HCAbs.

### SUMMARY OF THE DISCLOSURE

A first aspect of the disclosure provides a non-human animal cell comprising an antibody heavy chain locus comprising a plurality of human IgH V gene segments, one or more human IgH D gene segments and one or more human IgH J gene segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated; and
iii) there is substantially no expression of light chains and substantially no expression of non-human heavy chains

A second aspect of the disclosure provides a non-human animal comprising a plurality of cells according to the first aspect, wherein the animal is capable of expressing a repertoire of IgM and IgG heavy chain only antibodies.

A third aspect of the disclosure provides a method for producing a human heavy chain only antibody specific to a desired antigen, the method comprising immunising a non-human animal according to the second aspect of the disclosure with the desired antigen and recovering the antibody or its encoding nucleic acid or recovering a cell producing the antibody from the animal.

A fourth aspect of the disclosure provides a method of producing a pharmaceutical composition comprising an antibody specific to a desired antigen and a pharmaceutically acceptable carrier or other excipient, the method comprising immunising a non-human animal according to the second aspect, with the desired antigen and recovering the antibody and formulating the antibody with the pharmaceutically acceptable carrier or other excipient.

### DESCRIPTION OF THE FIGURES

**Figure 1** provides an overview of the steps for humanisation of the chromosome 12 IgH constant region in embryonic stem cells (mESCs) of a transgenic mouse according to the disclosure by recombinase-mediated cassette exchange (RMCE).
**Figure 2** provides an overview of a mouse Ig heavy chain locus on chromosome 12, containing a large fragment of the human Ig heavy chain locus (in 5' to 3' order) a set of 41 V gene segments, a set of D gene segments, and a set of 6 J gene segments, upstream of genes encoding a set of heavy chain constant regions from Cµ to Cα.
**Figure 3** illustrates the deletion of 200kb of endogenous mouse Lambda locus to generate Lambda knockout.
**Figure 4** provides an overview of the method of engineering a mouse Ig heavy chain locus according to the disclosure, requiring a first deletion of CH1 in Cµ and a subsequent second deletion of CH1 in Cγ1.
**Figure 5** shows a strategy for achieving the CC1-3 Cµ CH1 deletion.
**Figure 6** shows a strategy for generation of alleles with a partial deletion of the Cγ1 CH1.
**Figure 7** shows a strategy for generation of alleles with a full deletion of the Cγ1 CH1 and CH1 to hinge intron.
**Figure 8** illustrates the Vega2-28 Cγ1 locus (**8a**), Vega2-31 Cγ1 locus (**8b**) and Vega2-32 Cγ1 locus (**8c**).

### DETAILED DESCRIPTION

In a first aspect, the present disclosure relates to a non-human animal cell comprising an antibody heavy chain locus comprising a plurality of human IgH V gene segments, one or more human IgH D gene segments and one or more human IgH J gene segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated; and
iii) there is substantially no expression of light chains and substantially no expression of non-human heavy chains.

As used herein, the terms "human heavy VDJC DNA", "human heavy chain VDJC DNA", "human heavy VDJC region" or "human heavy VDJC" refer to DNA encoding for the V (Variable), D (Diversity) and J (Joining) gene segments (also referred to as "segments"), and the C (constant) genes (also referred to as "constant regions").

As used herein, the term "locus" refers to a specific position on a chromosome where a gene, gene segment or gene region comprising multiple gene segments is located.

As used herein, the terms "VDJ region" or "V(D)J region" refer to a DNA encoding for the V, D and J gene segments for the heavy chain, and V and J gene segments for the light chain. In an embodiment, the cell is homozygous for said antibody heavy chain locus.

"Endogenous" as used herein indicates that the gene, region (e.g. variable or constant region) or locus is a of gene, region or locus that is normally found in the vertebrate or cell (as opposed to an exogenous gene, region or locus whose sequence is not normally found in such a vertebrate or cell, e.g. a human sequence). For example, the endogenous constant region can be those encoded by the wild-type genome of the non-human vertebrate or cell. Therefore, in an example wherein the vertebrate is a mouse, the endogenous constant region would be a mouse constant region.

The term "substantially no expression" as used herein means essentially none (e.g. less than 10, 5, 4, 3, 2, 1 or 0.5%, or 0%) of the antibody chain (e.g. kappa or lambda light chain or non-human heavy chain V, D, J or C segments) is expressed (e.g. no endogenous light chain expressed and no non-human heavy chains expressed). In a particular embodiment, no kappa or lambda light chain is expressed. This can be determined, for example, at the antibody chain (protein) level by assessing the antibody repertoire produced by the non-human animal or at the nucleotide level by assessing mRNA transcripts of antibody chain loci, e.g. using RACE. In an embodiment, there is no expression of any light chains and no expression of any non-human heavy chains in a non-human animal cell of the invention.

A non-human animal cell according to the disclosure produces fully human heavy chain-only antibodies (HCAbs) that are advantageous in a number of ways, as described in more detail below. Said non-human animal cell is capable of producing antibodies of both IgM and IgG isotypes. The (full or partial) inactivation of the CH1 domains in Cµ and Cγ1 allows for the preservation of class switching from IgM to IgG and enables a natural affinity maturation process, resulting in greater diversity of fully human antibodies. Furthermore, the presence of both IgM and IgG heavy chain-only populations in the non-human animal allows differentiation between those that are somatically hypermutated and those that are not. It is possible to select only the IgG populations which are more likely to have high binding affinity for a given antigen of interest. Furthermore, the absence of any light chain sequence in the HCAb overcomes issues relating to residual pairing of light chains, e.g. with ΔCH1 IgM/ ΔCH1 IgG heavy chains, or with other isotypes, during B-cell proliferation and during somatic mutation. Transgenic non-human animals according to the disclosure are capable of producing fully human serum antibodies, i.e. they make human polyclonal serum in response to immunisation, which can be directly analysed with assays set up to interrogate human IgG1 response.

### Antibodies, variable domains, and specific antigen-binding

The term "antibody" herein includes monoclonal antibodies (including full length antibodies which have an immunoglobulin Fc region), antibody compositions with polyepitopic specificity, multi-specific antibodies (e.g. bispecific antibodies), and single-chain molecules, as well as antibody fragments.

The terms "antibody", "heavy chain-only antibody (HCAb)" and "H2 antibody" are used interchangeably herein. For the avoidance of doubt, all antibodies according to the present disclosure are heavy chain-only antibodies (HCAbs) that do not comprise functional light chain variable or functional light chain constant regions.

Antibodies and polypeptide domains herein are fully human antibodies generated directly from transgenic non-human animals whose genomes have been engineered to contain human variable region gene segments and human constant region genes. Accordingly, an antibody of the invention is a human antibody comprising multiple human heavy chain variable regions and human heavy chain constant regions. The human heavy chain constant regions are modified to inactivate the CH1 regions in the Cµ and Cγ gene segments.

An antibody variable domain provides a binding site for antigen. Recognition between an antibody and its cognate antigen may be referred to as specific binding, contrasting with nonspecific binding whereby an antibody or other polypeptide binds non-target molecules through relatively low affinity interactions. Antigen-binding of a variable domain is via contact between the antigen and one or (usually) multiple residues in the CDRs (HCDRs or LCDRs). One or more FR residues of the variable domain may also make contact with the antigen. The region of the antigen bound by the antibody is referred to as its epitope. The region of the antibody which binds the antigen is referred to as its paratope.

A variable domain or binding site that "specifically binds to" or is "specific for" a particular antigen or epitope may be one that binds to that particular antigen or epitope without substantially binding to other antigens or epitopes. For example, binding to the antigen or epitope is specific when the antibody binds with a KD of 1 mM or less, e.g. 100 mM or less, 10 pM or less, 1 pM or less, 100 nM or less, e.g. 10 nM or less, 1 nM or less, 500 pM or less, 100 pM or less, or 10pM or less. The binding affinity (K_{D}) can be determined using standard procedures as will be known by the skilled person, e.g., binding in ELISA and/or affinity determination using surface plasmon resonance (SPR) (e.g., Biacore^{™}, Proteon^{™} or KinExA^{™} solution phase affinity measurement which can detect down to fM affinities (Sapidyne Instruments, Idaho)). In one embodiment, SPR is carried out at 25°C. In another embodiment, the SPR is carried out at 37°C. In one example, the SPR is carried out at physiological pH, such as about pH 7 or at pH 7.6 (e.g. using Hepes buffered saline at pH 7.6 (also referred to as HBS-EP)). In one example, the SPR is carried out at a physiological salt level, e.g., 150 mM NaCl. In one example, the SPR is carried out at a detergent level of no greater than 0.05% by volume, e.g., in the presence of P20 (polysorbate 20; eg, Tween-20TM) at 0.05% and EDTA at 3mM. The SPR may be carried out at 25°C or 37°C in a buffer at pH 7.6, 150 mM NaCl, 0.05% detergent (eg, P20) and 3 mM EDTA. The buffer can contain 10 mM Hepes. In one example, the SPR is carried out at 25°C or 37°C in HBS-EP. HBS-EP is available from Teknova Inc (California; catalogue number H8022).

### Bispecific antibodies

An HCAb is useful in a bispecific or multispecific antigen-binding format. Such a format may comprise first and second variable domains, wherein the first variable domain specifically binds a first antigen or epitope and the second variable domain specifically binds a second antigen or epitope. The first and second variable domains may have amino acid sequences that differ from one another, for binding to different first and second antigens/epitopes respectively. The different epitopes may be epitopes of one antigen or of different antigens. Thus, for example the first variable domain may specifically bind antigen A (and not antigen B) and the second variable domain may specifically bind antigen B (and not antigen A).

There are advantages to providing an antigen-binding site within a variable domain which is expressible as part of a single polypeptide chain, since a bispecific antibody can be generated through the association (e.g., following co-expression) of two such polypeptides comprising different variable domains (and thus different antigen-binding sites). This contrasts with the more complex generation of a typical four-chain bispecific antibody which comprises two different heavy-light chain pairs and thus 4 different polypeptide chains.

### Engineering the lgH variable region

A non-human animal genome may be engineered to contain a plurality of human heavy chain V gene segments, one or more human heavy chain D gene segments and one or more human heavy chain J gene segments, for expression of a human VH domain. It may contain a full set of all human heavy chain V, D and J gene segments. The human VDJ region may be inserted upstream of a constant region, for production of VH domains linked to a constant region. As discussed below, the constant region is a human constant region.

Recombination of V(D)J gene segments generates combinatorial diversity in each variable domain. With the inclusion of a full set of human heavy chain gene segments, the full combinatorial diversity of human variable domains can be incorporated into a transgenic animal platform. Affinity maturation of these variable domains then proceeds through the natural *in vivo* processes of somatic hypermutation and selection, providing an extensive and diverse sequence repertoire from which antigen-specific variable domains with desirable properties may be identified and their sequences recovered.

Methods of generating transgenic animals having genomes comprising all or part of human immunoglobulin loci are well known in this technical field. Such animals have been used to discover and produce several antibodies comprising human variable domains which are currently on the market as pharmaceutical products. Methods of engineering the non-human animal genome to contain human immunoglobulin gene segments are described for example in WO2011/004192 (Genome Research Limited), which is incorporated herein by reference. Examples of transgenic non-human animals include the transgenic mouse described in WO2011/004192, VelociMouse^{®}, OmniMouse^{®}, Omnirat^{®}, XenoMouse^{®}, HuMab mouse^{®} and MeMo Mouse^{®}.

In an embodiment, the non-human animal is a rodent, preferably a mouse or a rat. Preferably, the transgenic non-human animal is a transgenic rodent, preferably a transgenic mouse or a transgenic rat.

In an embodiment, the inserted human DNA comprises at least 50% of the human heavy chain variable (V) genes, such as at least 60%, at least 70%, at least 80%, at least 90%, at least 95%, at least 99%, and preferably all of the human V genes.

In an embodiment, the inserted human DNA comprises at least 50% of the human heavy chain diversity (D) genes, such as at least 60%, at least 70%, at least 80%, at least 90%, at least 95%, at least 99%, and preferably all of the human D genes.

In an embodiment, the inserted human DNA comprises at least 50% of the human heavy chain joining (J) genes, such as at least 60%, at least 70%, at least 80%, at least 90%, at least 95%, at least 99%, and preferably all of the human J genes

The inserted human genes may be derived from the same individual or different individuals, or be synthetic or represent human consensus sequences.

In one embodiment the human heavy chain locus inserted into the non-human mammal contains the full repertoire of human V, D and J gene segments. This total inserted human heavy chain genetic material is referred to herein as the "human IgH VDJ region" or "human heavy VDJ region", and comprises DNA from a human genome that encodes all the exons encoding human V, D and J portions and suitably also the associated introns.

In an embodiment, the inserted human IgH VDJ region comprises, in germline configuration, all of the V, D and J gene segments and intervening sequences from a human.

*In situ* targeting of the human immunoglobulin locus DNA to the endogenous immunoglobulin loci of the non-human animal, in contrast with random insertion, facilitates the correct and precise regulation of the immune response. Transgenic animals of the present invention can thus display a robust immune response, including somatic hypermutation and affinity maturation of antibody variable domains, following exposure to an immunogenic composition comprising target antigen.

In one embodiment, 800-1000kb of the human IgH VDJ region is inserted into the non-human mammal IgH locus, and in one embodiment a 940, 950 or 960 kb fragment is inserted. Suitably this includes bases 105,400,051 to 106,368,585 from human chromosome 14 (all coordinates refer to NCBI36 for the human genome, ENSEMBL Release 54 and NCBIM37 for the mouse genome, relating to mouse strain C57BL/6J).

In one embodiment the inserted IgH human fragment consists of bases 105,400,051 to 106,368,585 from chromosome 14. In one embodiment the inserted human heavy chain DNA, such as DNA consisting of bases 105,400,051 to 106,368,585 from chromosome 14, is inserted into mouse chromosome 12 between the end of the mouse J4 gene segment and the Eµ region, suitably between coordinates 114,667,091 and 114,665,190, suitably at coordinate 114,667,091.

According to the present disclosure, there is substantially no expression of non-human heavy chains. In an embodiment, there is no expression of any non-human heavy chain V, D, or J gene segments. An example methodology for inactivating the endogenous VDJ sequences is provided in Example 1 below.

In an embodiment, the endogenous non-human animal heavy chain V, D, and J gene segments are inactivated to render these gene segments non-functional. This can be achieved by introducing a loss-of-function mutation, full or partial deletion, or introduction of an inactivating mutation. In a preferred embodiment, the endogenous non-human animal heavy chain V, D, and J gene segments are inactivated by introducing a full deletion. In another preferred embodiment, the endogenous non-human animal heavy chain V, D, and J gene segments are inactivated by introducing a partial deletion.

In one embodiment, inactivation is achieved by inversion of all or part of the non-human animal VDJ gene segments, optionally by insertion of one or more site specific recombinase sites into the genome and then use of these sites in recombinase-mediated excision or inversion of all or a part of the non-human animal Ig locus. In one embodiment, a double inversion, may be employed, the first to move the VDJ gene segments away from the endogenous locus and then a more local inversion which puts them in the correct orientation. In one embodiment, a single IoxP site is used to invert the non-human animal VDJ gene segments to a centromeric locus or telomeric locus.

In an embodiment, one or more of the inverted endogenous heavy chain J gene segments are partially or fully deleted.

In an alternative embodiment, the endogenous heavy chain VDJ regions are inactivated by full or partial deletion of the endogenous heavy chain V and/or J gene segments.

### Engineering the lgH constant region

In addition to the heavy chain variable region, the genome of the non-human animal encodes the heavy chain constant region of an antibody described herein. The locus comprising the variable region gene segments further comprises a constant region gene or genes, e.g. a heavy chain constant region comprising CH1, CH2 and/or CH3 domains, and a hinge region, located between CH1 and CH2, for added flexibility. According to the present disclosure, the constant region is a fully human IgH constant region, i.e. the domains are of human origin and all constant region sequences, including interdomain region sequences (e.g. hinge region sequences) and regulatory control sequences (e.g. 5' enhancer sequences, switch region sequences, PolyA, TM1 and TM2 sequences, 3' locus control region (LCRT) sequences) are fully human sequences.

The non-human animal genome may be engineered to contain the full repertoire of human constant region genes: IgM, IgD, IgG1, IgG2, IgG3, IgG4, IgE, lgA1 and IgA2, e.g. by insertion of a fragment of human genomic DNA containing these genes.

Intergenic and regulatory elements of the human immunoglobulin locus, including enhancers, are preferably also included.

In an embodiment, the inserted human heavy chain DNA is the complete human heavy chain VDJC region, including all intergenic and regulatory elements of the human IgH locus.

Sequences of exemplary human constant regions are provided in Table A of WO2020/049128 (Kymab), the content of which is incorporated herein by reference.

According to the present disclosure, there is substantially no expression of non-human heavy chains. Therefore, there is substantially no expression of the endogenous IgH constant region. In an embodiment, there is no expression of any non-human IgH constant region.

In an embodiment, the endogenous non-human animal IgH C region is inactivated to render this region non-functional. This can be achieved by introducing a loss-of-function mutation, full or partial deletion, or introduction of an inactivating mutation, such as inversion as described above. In an embodiment, the endogenous non-human animal IgH C region sequence is excised and replaced by the insertion of the human IgH C region sequence.

According to the present disclosure, the human heavy chain constant region comprises a Cµ gene segment, and a Cγ1 gene segment, and optionally also one or more of an alpha, delta, epsilon and other gamma C gene segments. In an embodiment, the constant region comprises all of the human Cµ, Cδ, Cε, Cγ1, Cγ2, Cγ3 and Cα gene segments.

In an embodiment, the Cµ gene segment is positioned is upstream of the Cγ1 gene segment. In an embodiment, the gene segments of the C region are in germline configuration of C segments found in wildtype (human and mouse) IgH locus, i.e., in 5' to 3' orientation, Cµ, Cδ, Cγ3, Cγ1, Cα1, Cγ2, Cγ4, Cε and Cα2.

According to the present disclosure, the CH1 domain is inactivated in both of the heavy chain Cµ and Cγ1 gene segments, such that there is no expression of a CH 1 domain in the Cµ gene segment and/or the Cγ1 gene segment.

The CH1 domain can be inactivated in the Cµ and Cγ1 gene segments by any suitable means, including by introduction of a loss-of-function mutation, partial or full deletion of a nucleotide sequence encoding at least part of the CH1 domain or by introduction of an inactivating mutation introducing a stop codon in CH1 domain of the Cµ and/or Cγ1 gene segments.

As used herein, the term "loss-of-function mutation" can refer to any genetic alteration, compared to the wild-type gene, resulting in inactivation of the gene segment (i.e. loss of transcriptional activity and/or failure to express functional protein). Loss of function-related alterations may encompass the entire gene locus or may be located within the gene sequence or in regulatory or promoter sequences. Loss of function-related alterations include, but are not limited to, point mutations, insertions, deletions, frame-shift mutations and translocations, resulting in gene inactivation or a null allele genotype.

Inactivation of the CH1 domain in the Cµ and Cγ1 gene segments many be achieved by full or partial deletion of the relevant gene segments, such that there is no expression of CH1 in Cµ and Cγ1 gene segments.

In an embodiment, CH1 is inactivated in the Cµ gene segment by partial deletion of the CH1 domain. In an embodiment, CH1 is inactivated in the Cγ1 gene segment by partial deletion of the CH1 domain, In an embodiment, CH1 is inactivated in both the Cµ and Cγ1 gene segments by partial deletion of both CH1 domains,

In an embodiment, the part of the CH1 domain that is deleted comprises all or part of the CH1 domain, preferably at least 50, 55, 60, 65, 70, 75, 80, 85, 90, 95, 99 or 100% of the entire nucleotide sequence encoding the CH1 domain.

In an embodiment, CH1 is inactivated in the Cµ gene segment by full deletion of the CH1 domain. In an embodiment, CH1 is inactivated in the Cγ1 gene segment by full deletion of the CH1 domain.

In an embodiment, CH1 is inactivated in both the Cµ and Cγ1 gene segments by full deletion of both CH1 domains.

As used herein, the term "full deletion" refers to deletion of the entire nucleotide sequence encoding the CH1 domain.

As used herein, the term "partial deletion" refers to deletion of less than 100% of the entire nucleotide sequence encoding the CH1 domain, wherein the deletion results in inactivation of the CH1 domain, such that there is no expression of a functional CH1 domain protein. In an embodiment, "partial deletion" refers to deletion of at least 50, 55, 60, 65, 70, 75, 80, 85, 90, 95, 96, 97, 98, or 99% of the entire nucleotide sequence encoding the CH1 domain.

In an embodiment, CH1 is inactivated in the Cµ gene segment by full deletion of the entire CH1 domain and CH1 is inactivated in the Cγ1 gene segment by partial deletion of the CH1 domain.

In an embodiment, CH1 is inactivated in the Cµ gene segment by partial deletion of the CH1 domain and CH1 is inactivated in the Cγ1 gene segment by full deletion of the entire CH1 domain.

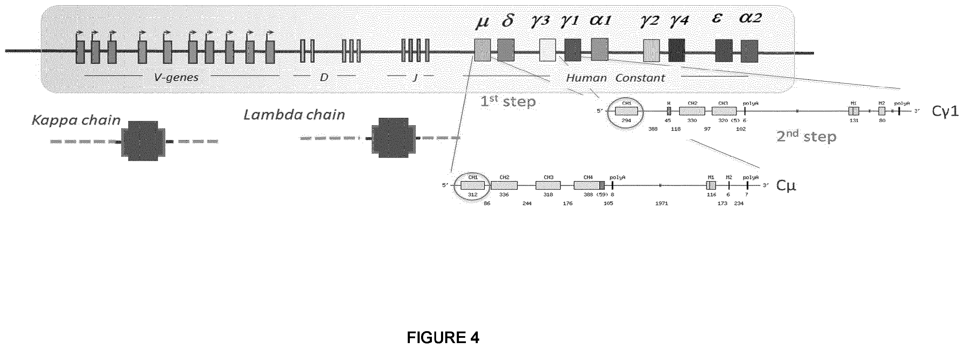

Inactivation of the CH1 domain in the Cµ and Cγ1 gene segments may be achieved in a single or multistep process. In an embodiment, inactivation of the gene segments may be achieved in a two-step process comprising a first partial of full deletion of CH1 in Cµ and a subsequent second partial of full deletion in Cγ1. This is illustrated in Figure 4 and described in Example 2 below.

Alternatively, the CH1 domain can be inactivated in the Cµ and Cγ1 gene segments in a two-step process comprising a first deletion of CH1 in Cγ1 and a subsequent second deletion in Cµ.

Alternatively, the CH1 domain can be inactivated in both the Cµ and Cγ1 gene segments simultaneously in a single step, e.g. in a multiplex modification.

The CH1 domain is retained in some or all of the other constant region gene segments. In an embodiment, the heavy chain constant region encodes a CH1 domain in each of the Cδ, Cε, Cγ2, Cγ3 and Cα gene segments.

Therefore, in one embodiment, the disclosure relates to a non-human animal cell comprising an antibody heavy chain locus comprising a plurality of human IgH V gene segments, one or more human IgH D gene segments and one or more human IgH J genes segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated by full deletion of both CH1 domains; and
iii) there is no expression of light chains and no expression of non-human heavy chains.

All the technical features disclosed herein apply to this embodiment.

In another embodiment, the disclosure relates to a non-human animal cell comprising an antibody heavy chain locus comprising a plurality of human IgH V gene segments, one or more human IgH D gene segments and one or more human IgH J genes segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated by partial deletion of both CH1 domains; and
iii) there is no expression of light chains and no expression of non-human heavy chains.

All the technical features disclosed herein apply to this embodiment.

In another embodiment, the disclosure relates to a non-human animal cell comprising an antibody heavy chain locus comprising a plurality of human IgH V gene segments, one or more human IgH D gene segments and one or more human IgH J genes segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain is inactivated by full deletion of the CH1 domain and the Cγ1 CH1 domain is inactivated by partial deletion of the CH1 domain; and
iii) there is no expression of light chains and no expression of non-human heavy chains.

All the technical features disclosed herein apply to this embodiment.

### Inactivation of CH1 in Cu

SEQ ID NO. 7, referred to herein as the CC1-3 allele, is an exemplary sequence of the Cµ gene segment wherein the CH1 region is inactivated by full deletion of the CH1 gene. See Example 2 below for a detained description of a methodology for generating this allele.

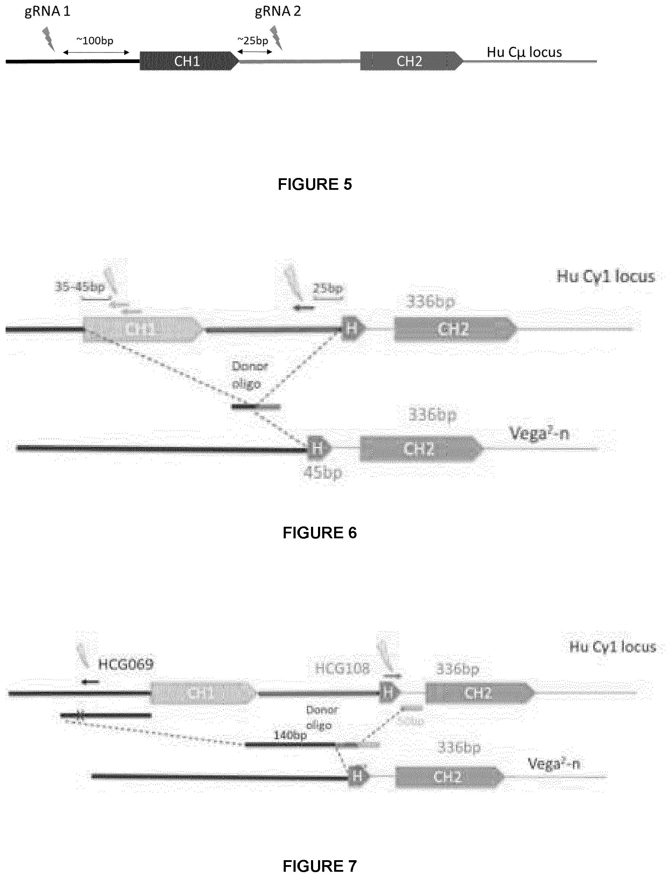

An overview of a strategy for inactivation of CH1 in Cµ is provided in Figure 5.

### Inactivation of CH1 in Cγ1

SEQ ID Nos. 11 and 12, referred to herein as the Vega2-8 allele and Vega 2-15 allele respectively, are exemplary sequences of Cγ1 gene segments comprising a partial deletion of CH1. The generation of these alleles is described in detail in Example 2 below, and illustrated in Figure 6 and 7, respectively.

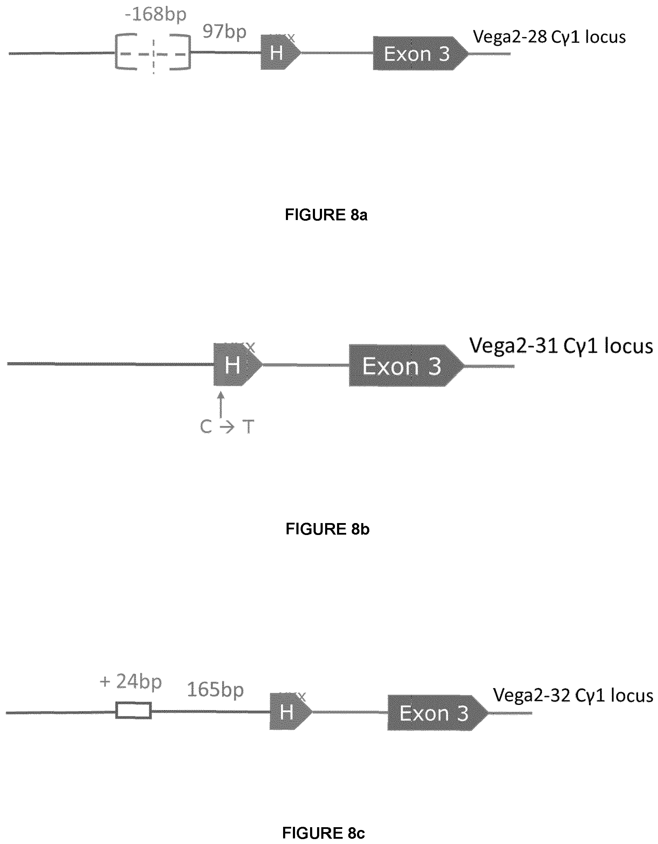

SEQ ID NO. 16, referred to herein as the Vega2-28 allele, is an exemplary sequence of the Cγ1 gene segment comprising full deletion of CH1 (shown in Figure 8a and described in detail in Example 2 below).

In an embodiment, the Cγ1 hinge region is engineered to comprise a H237Y mutation. The presence of a single nucleotide polymorphism (SNP) has been shown to substantially improve fragmentation resistance in the presence of H₂O₂ of IgG1 - see S Suzuki et al. (Nature Scientific Reports (2018) 8:17253), the content of which is incorporated herein by reference.

SEQ ID NO. 17, referred to herein as Vega2-31 allele, is an exemplary sequence of the Cγ1 gene segment comprising full deletion of CH1 and incorporation of donor sequence with a C to T point mutation that results in a non-deleterious histidine to tyrosine change (H237Y) in the IgG1 coding sequence (SEQ ID NO. 18; described in detail in Example 2 below).

SEQ ID NO. 19, referred to herein as Vega2-32 allele, is an alternative exemplary sequence of a Cγ1 gene segment comprising full deletion of CH1 (described in detail in Example 2 below).

### Inactivation of the endogenous Ig light chain loci

According to the present disclosure, there is substantially no expression of light chains in the non-human animal cell of the invention, i.e. the endogenous light chain is inactivated to prevent expression of a functional Ig light chain.

In an embodiment, there is no expression of any lambda or kappa light chains.

In an embodiment, all endogenous lambda and/or kappa V and/or J genes are inactivated, preferably by introduction of one or more loss-of-function mutations within, or full or partial deletion of, the endogenous lambda and kappa light chain loci.

In an embodiment, all endogenous lambda and/or kappa V and J genes are inactivated.

In an embodiment, the endogenous lambda locus retains JC1 and/or JC3. In an embodiment, the endogenous lambda locus retains JC1 and JC3. In an embodiment, all endogenous kappa V genes are deleted. In another embodiment, the endogenous lambda locus retains JC1 and JC3 and all endogenous kappa V genes are deleted.

By turning off light chain expression, heavy chains are expressed in the absence of light chain expression and thus there is no possibility for residual pairing of light chains, either with ΔCH1 IgM/ ΔCH1 IgG heavy chains, or with other isotypes (e.g. IgD) during B-cell proliferation and during somatic mutation and selection of heavy chains following antigen or epitope immunisation. Thus, heavy chain production is essentially exclusively the production of H2 antibody-type heavy chains, wherein the variable regions bind antigen in the absence of a light chain variable region partner.

Such H2 antibodies can be a good source for antibody single variable domain sequences (e.g. human VH single variable domains when the heavy chain loci comprise human variable regions). Thus, the *in vivo* antibody production machinery of the non-human vertebrate or cell of the invention can be dedicated to H2 antibody production without any significant 4-chain antibody (H2L2) production hampering the extent of H2 antibody response following immunisation and ultimately the desirable levels of H2 that are obtainable.

Example methodologies for inactivating the endogenous kappa and lambda genes are provided in Example 1 below. Furthermore, Example 5 of WO2020/049128 describes inactivation of endogenous kappa and lambda light chain loci in transgenic animals, resulting in generation of fully human VH-only antibodies. The content of this publication is incorporated by reference herein.

Therefore, an embodiment of the present disclosure provides a non-human animal cell comprising an antibody heavy chain locus comprising a plurality of human IgH V gene segments, one or more human IgH D gene segments and one or more human IgH J gene segments upstream of all or part of a human heavy constant (C) region and forming a human heavy VDJC region, wherein:
i) the human heavy VDJC DNA is inserted upstream of, downstream of or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated; and
iii) the non-human animal cell does not express any endogenous or exogenous kappa or lambda light chains and does not expression any endogenous or non-human heavy chains.

A non-human animal cell according to the present disclosure produces antibodies of both IgG and IgM isotypes and is capable of undergoing isotype switching. In one aspect, the invention therefore relates to a non-human animal cell according to the present disclosure, wherein said cell produces antibodies of both IgG and IgM isotypes and is capable of undergoing isotype switching. Typically, said cell produces IgM antibodies which can mature, leading the cell to undergo class-switching to produce other isotype such as IgG, IgA or IgE, preferably IgG.

A second aspect of the disclosure provides a non-human animal comprising a plurality of cells according to the first aspect, wherein the animal is capable of expressing a repertoire of IgM and IgG heavy chain only antibodies.

### Immunisation

Further aspects of the disclosure are directed to the use of non-human animals described herein for producing human HCAbs that specifically bind target antigens. A HCAb according to the present invention may be produced by exposing a non-human animal as described herein to immunogenic stimulation with the target antigen.

Accordingly, a third aspect of the disclosure provides a method for producing a human HCAb specific to a desired antigen, the method comprising immunising a non-human animal according to the second aspect of the disclosure with the desired antigen and recovering the antibody or its encoding nucleic acid or recovering a cell producing the antibody from said animal.

In an embodiment, the antigen is selected from a human cytokine, growth factor, hormone, enzyme and a serum protein. In an embodiment, the antigen is a multi-subunit human protein antigen.

The non-human animal can be challenged with the target antigen, and lymphatic cells (such as B cells) can then recovered from animals that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. Nucleic acid encoding an antibody heavy chain can be isolated from lymphocytes.

Alterations to nucleic acid encoding the antibody heavy chain region may be performed, such as mutation of residues and generation of variants. There are many reasons why it may be desirable to create variants, which include optimising the sequence for large-scale manufacturing, facilitating purification, enhancing stability or improving suitability for inclusion in a desired pharmaceutical formulation. Protein engineering work can be performed at one or more chosen residues in the sequence, e.g., to substituting one amino acid with an alternative amino acid (optionally, generating variants containing all naturally occurring amino acids at this position, with the possible exception of Cys and Met), and monitoring the impact on function and expression to determine the best substitution. It is in some instances undesirable to substitute a residue with Cys or Met, or to introduce these residues into a sequence, as to do so may generate difficulties in manufacturing - for instance through the formation of new intramolecular or intermolecular cysteine-cysteine bonds. Where a lead candidate has been selected and is being optimised for manufacturing and clinical development, it will generally be desirable to change its antigen-binding properties as little as possible, or at least to retain the affinity and potency of the parent molecule. However, variants may also be generated in order to modulate key antibody characteristics such as affinity, cross-reactivity or neutralising potency.

In an embodiment, the method further comprises cloning the encoding nucleic acid into a recombinant host cell e.g. a CHO, HEK293, Cos or yeast cell, culturing the cell for expression of the heavy chain antibody, and recovering and purifying the antibody from the cell or culture medium.

In an embodiment, the method further comprises formulating the antibody or antibody chain with a pharmaceutically acceptable carrier or other excipient to produce a pharmaceutical composition.

### Pharmaceutical compositions

A fourth aspect of the disclosure provides a method of producing a pharmaceutical composition comprising an antibody specific to a desired antigen and a pharmaceutically acceptable carrier or other excipient, the method comprising immunising a non-human animal according to the second aspect, with the desired antigen and recovering the antibody and formulating the antibody with the pharmaceutically acceptable carrier or other excipient.

In an embodiment, the method further comprises packaging the composition in a sterile container.

In an embodiment, the method further comprises producing a kit comprising combining the package with a label or instructions indicating use of the antibody composition for human medical use. In an embodiment, the label or instructions comprises a medicament batch number and/or marketing authorisation number, e.g. an EMA or FDA marketing authorisation number.

Antibodies and their encoding nucleic acid according to the present invention may be provided in isolated form and/or in solution, e.g., aqueous solution.

The invention further provides a composition (e.g., a pharmaceutical composition or a composition for medical use) comprising a HCAb obtained or obtainable by a method of the invention as disclosed herein.

Antibodies may be monoclonal or polyclonal, but are preferably provided as monoclonal antibodies for therapeutic use. They may be provided as part of a mixture of other antibodies, optionally including antibodies of different binding specificity.

Antibodies according to the invention, and encoding nucleic acids, will usually be provided in isolated form. Thus, the antibodies and nucleic acids may be provided purified from their natural environment or their production environment. Isolated antibodies and isolated nucleic acid will be free or substantially free of material with which they are naturally associated, such as other polypeptides or nucleic acids with which they are found in vivo, or the environment in which they are prepared (e.g., cell culture) when such preparation is by recombinant DNA technology in vitro. Optionally, an isolated antibody or nucleic acid (1) is free of at least some other proteins with which it would normally be found, (2) is essentially free of other proteins from the same source, e.g., from the same species, (3) is expressed by a cell from a different species, (4) has been separated from at least about 50 percent of polynucleotides, lipids, carbohydrates, or other materials with which it is associated in nature, (5) is operably associated (by covalent or noncovalent interaction) with a polypeptide with which it is not associated in nature, or (6) does not occur in nature.

Antibodies or nucleic acids may be formulated with diluents or adjuvants and still for practical purposes be isolated - for example they may be mixed with carriers if used to coat microtitre plates for use in immunoassays, and may be mixed with pharmaceutically acceptable carriers or diluents when used in therapy. Other active ingredients may also be included in therapeutic preparations. Antibodies may be glycosylated, either naturally in vivo or by systems of heterologous eukaryotic cells such as CHO cells, or they may be (for example if produced by expression in a prokaryotic cell) unglycosylated. The invention encompasses antibodies having a modified glycosylation pattern. In some applications, modification to remove undesirable glycosylation sites may be useful, or e.g., removal of a fucose moiety to increase ADCC function. In other applications, modification of galactosylation can be made in order to modify CDC.

Typically, an isolated product constitutes at least about 5%, at least about 10%, at least about 25%, or at least about 50% of a given sample. An antibody may be substantially free from proteins or polypeptides or other contaminants that are found in its natural or production environment that would interfere with its therapeutic, diagnostic, prophylactic, research or other use.

An antibody may have been identified, separated and/or recovered from a component of its production environment (e.g. naturally or recombinantly). The isolated antibody may be free of association with all other components from its production environment, e.g., so that the antibody has been isolated to an FDA-approvable or approved standard. Contaminant components of its production environment, such as that resulting from recombinant transfected cells, are materials that would typically interfere with research, diagnostic or therapeutic uses for the antibody, and may include enzymes, hormones, and other proteinaceous or non-proteinaceous solutes. In some embodiments, the antibody will be purified: (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of a spinning cup sequenator, or (3) to homogeneity by SDS-PAGE under non reducing or reducing conditions using Coomassie blue or silver stain. Isolated antibody includes the antibody in situ within recombinant cells since at least one component of the antibody's natural environment will not be present. Ordinarily, however, an isolated antibody or its encoding nucleic acid will be prepared by at least one purification step.

The polypeptides comprising HCAbs of the present invention, or their encoding nucleic acids, may be formulated for the desired route of administration to a patient, e.g., in liquid (optionally aqueous solution) for injection. Compositions may comprise a polypeptide or nucleic acid in combination with medical injection buffer and/or with adjuvant. Various delivery systems are known and can be used to administer the pharmaceutical composition of the invention. Methods of introduction include, but are not limited to, intradermal, intramuscular, intraperitoneal, intravenous, subcutaneous, intranasal, epidural, and oral routes.

The composition may comprise a diluent, excipient or carrier. When the composition is a pharmaceutical composition or a composition for medical use, the diluent, excipient or carrier is pharmaceutically acceptable. "Pharmaceutically acceptable" refers to approved or approvable by a regulatory agency of the USA Federal or a state government or listed in the U.S. Pharmacopeia or other generally recognized pharmacopeia for use in animals, including humans.

A "pharmaceutically acceptable carrier, excipient, or adjuvant" refers to a carrier, excipient, or adjuvant that can be administered to a subject, together with an agent, e.g. any antibody described herein, and which does not destroy the pharmacological activity thereof and is nontoxic when administered in doses sufficient to deliver a therapeutic amount of the agent. Compositions comprising polypeptides or nucleic acids described herein may be contained in a sterile container in vitro. A composition may be in a bag or other medical container connected to an IV syringe. It may be within a phial, syringe or an injection device. In an example, a kit is provided comprising the antibody, polypeptide or nucleic acid, plus packaging and instructions for use in a therapeutic method.

The disclosure provides therapeutic compositions comprising polypeptides comprising antibody heavy chains as described herein. Therapeutic compositions comprising nucleic acid encoding such polypeptides are also provided. Encoding nucleic acids are described in more detail elsewhere herein and include DNA and RNA, e.g. mRNA. In therapeutic methods described herein, use of nucleic acid encoding the antibody, and/or of cells containing such nucleic acid, may be used as alternatives (or in addition) to compositions comprising the antibody itself. Cells (e.g., human cells, e.g., human lymphocytes) containing nucleic acid encoding the antibody, optionally wherein the nucleic acid is stably integrated into the genome, thus represent medicaments for therapeutic use in a patient. Cells expressing CARs, e.g., CAR-T cells, are an example. Alternatively, nucleic acid encoding an antibody of the present invention may be introduced into human B lymphocytes, optionally B lymphocytes derived from the intended patient and modified ex vivo. Optionally, memory B cells are used. Administration of cells containing the encoding nucleic acid to the patient provides a reservoir of cells capable of expressing the antibody, which may provide therapeutic benefit over a longer term compared with administration of isolated nucleic acid or isolated antibody.

It will be understood that particular embodiments described herein are shown by way of illustration and not as limitations of the invention. The principal features of this invention can be employed in various embodiments without departing from the scope of the invention. Those skilled in the art will recognise, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context. Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

### SEQUENCES

**SEQ ID NO. 1: gRNA ID # HCG013**
   GATGCCACTGGGGACGCCAA
**SEQ ID NO. 2: gRNA ID # HCG014**
   AAGTCCCTTGGCGTCCCCAG
**SEQ ID NO. 3: gRNA ID # HCG015**
   GGAGCCGGCTGAGAGAAGTT
**SEQ ID NO. 4: gRNA ID # HCG016**
   TGGAGATAATCTGTCCTAA (S
**SEQ ID NO. 5: gRNA ID # HCG062**
   TCACTACTTGCGTCCCGCTG
**SEQ ID NO. 6: gRNA ID # HCG066**
   GCCCAGCCACCGGGACAGAG
**SEQ ID NO. 7: Nucleotide sequence of Cµ ΔCH1 CC1-3 allele** 
CCTGAGGCCTCACCACGGCCCCGCCACCCCTGATAGCCATGACAGTCTCTCTAGAGAGGGAAG
AGAGGGAGCCGAAGGGGGCGGGAGTGGCGGGCACCGGGCTGACACGTG
**SEQ ID NO. 8: gRNA ID # HCG104**
   GATTGGGAGTTACTGGAATC
**SEQ ID NO. 9: gRNA ID # HCG105**
   GCCCCCAGAGGTGCTCTTGG
**SEQ ID NO. 10: ssDNA donor template** 
CTGCGCCCTGGGCCCAGCTCTGTCCCACACCGCGGTCACATGGCACCACCTCTCTTGCAGAGC
CCAAATCTTGTGACAAAACTCACACATGCCCACCGTGCCCAGGTAAGCCAGCCCAGG
**SEQ ID NO. 11: Nucleotide sequence of human Cγ1 (partial) ΔCH1 Vega2-8 allele** 
CCTCCACCAAGGGCCCATCGGTCTTCCCCCTGGCACCCTCCTCCAATCCAGTAACTCCCAATCT
TCTCTCTGCAG
**SEQ ID NO. 12: Nucleotide sequence of human Cγ1 (partial) ΔCH1 Vega2-15 allele** 
CCTCCACCAAGGGCCCATCGGTCTTCCCCCTGGCACCCTCCTCCACTGCAGAGAGAAGATTGG
GAGTTACTG
**SEQ ID NO. 13: gRNA ID # HCG069**
   TGGGTTCTTAACTGTCCGCG
**SEQ ID NO. 14: gRNA ID # HCG108**
   CACACATGCCCACCGTGCCC
**SEQ ID NO. 15: ssDNA donor template** 
CCAGCCAGGCGCACACCCAATGCCCGTGAGCCCAGACACTGGACGCTGAACGTCGCGGACAGT
TAAGAACCCAcGGGCCTCTGCGCCCTGGGCCCAGCTCTGTCCCACACCGCGGTCACATGGCAC
CACCTCTCTTGCAGAGCCTAAATCTTGTGACAAAACTCACACCTGTCCTCCGTGCCCAGGTAAGC
CAGCCCAGGCCTCGCCCTCCAGCTCAAGGCGGGACAGGTGCCC
**SEQ ID NO. 16: Nucleotide sequence of human Cγ1 ΔCH1 Vega2-28 allele** 
GTCCCTGAGGTGGCACCGATGGCCAGAGCTGAGGCCAAGCTAGAGGCCCTGGACTGTGCTGAC
TCCCGGCAGACACAGAGCGCTGACCTGGCTGCCGAGCCCCGCCCCCGCTGAACGTCGCGGAC
AGTTAAGAACCCACGGGCCTCTGCGCCCTGGGCCCAGCTCTGTCCCACACCGCGGTCACATGG
CACCACCTCTCTTGCAGAGCCTAAATCTTGTGACAAAACTCACACCTGTCCTCCGTGCCCAG
**SEQ ID NO. 17: Nucleotide sequence of human Cγ1 ΔCH1 Vega2-31 allele** 
AGCCCAGACACTGGACGCTGAACGTCGCGGACAGTTAAGAACCCACGGGCCTCTGCGCCCTGG
GCCCAGCTCTGTCCCACACCGCGGTCACATGGCACCACCTCTCTTGCAGAGCCTAAATCTTGTG
ACAAAACTTACACCTGTCCTCCGTGCCCAG
**SEQ ID NO. 18: Amino acid sequence of IgG1 hinge region with point mutation (shown in bold):**
   EPKSCDKTYTCPPCP
**SEQ ID NO. 19: Nucleotide sequence of human Cγ1 ΔCH1 Vega2-32 allele**

CCAGCCAGGCGCACACCCAATGCCCGTGAGCCCAGACACTGGACGCTGAACCTCGCCCAGACA
CTGGACGCTGAACGTCGCGGACAGTTAAGAACCCAcGGGCCTCTGCGCCCTGGGCCCAGCTCT
GTCCCACACCGCGGTCACATGGCACCACCTCTCTTGCAGAGCCTAAATCTTGTGACAAAACTCA
CACCTGTCCTCCGTGCCCAG

The disclosure will now be further described by reference to the following non-limiting examples.

### EXAMPLES

### Example 1: Generation of transgenic mouse comprising fully human heavy chain and inactivated mouse lambda and kappa loci

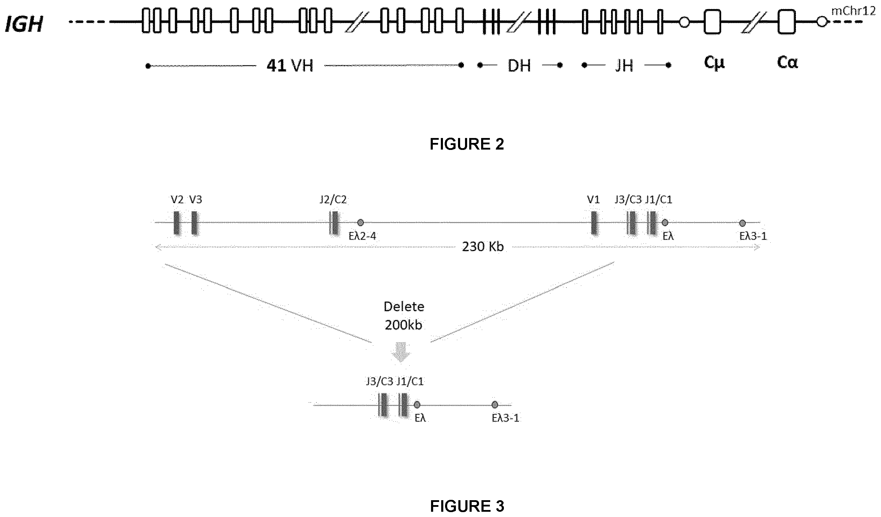

Human heavy chain V, D, J gene segments were inserted at the mouse heavy chain locus on chromosome 12, as illustrated in **Figure 2****.**

Transgenic animals of the invention comprising human IgH-VDJ can be generated using a site-specific recombination (SSR)-based technique called sequential RMCE (SRMCE), which allows continuous insertion of BAC inserts into the same locus. The methods summarised below for introducing human IgH VDJ genes and inactivating the endogenous VDJ are described in detail in Examples 1 and 2 of WO2011/004192 the content of which is incorporated herein in its entirety.

### Insertion of human VDJ gene segments

The method comprises the steps of:
1. insertion of DNA forming an initiation cassette (also called a landing pad herein) into the genome of a cell;
2. insertion of a first DNA fragment into the insertion site, the first DNA fragment comprising a first portion of a human DNA and a first vector portion containing a first selectable marker or generating a selectable marker upon insertion;
3. removal of part of the vector DNA;
4. insertion of a second DNA fragment into the vector portion of the first DNA fragment, the second DNA fragment containing a second portion of human DNA and a second vector portion, the second vector portion containing a second selectable marker, or generating a second selectable marker upon insertion;
5. removal of any vector DNA to allow the first and second human DNA fragments to form a contiguous sequence; and
6. iteration of the steps of insertion of a part of the human V(D)J DNA and vector DNA removal, as necessary, to produce a cell with all or part of the human VDJ or VJ region sufficient to be capable of generating a chimeric antibody in conjunction with a host constant region, wherein the insertion of at least one DNA fragment uses site specific recombination.

### Inactivation of endogenous VDJ

The endogenous VDJ sequences were inactivated through an inversion via chromosomal engineering as follows:
1. Targeting a "flip-over" cassette into a 5' region 10 to 40 megabases away from the endogenous VDJ. The flip-over vector comprises PB3'LTR - PGK promoter - HPRT mini gene 5' portion - loxP - puroΔTK - CAGGS promoter - PB3'LTR.
2. Transient CRE expression results in recombination between the loxP site in the "flip- over" cassette and the loxP site in the 5' modification. This 5' modification results from insertion of an odd number of BACs, after the 3' modification has been cured. The loxP sites are inverted relative to one another and therefore the described recombination event results in an. Cells with the correct inversion will be HAT-resistance since the HPRT mini-gene is reconstituted by a correct inversion.
3. A correct inversion also leaves two transposon structures flanking the "flip-over" cassette and the 5' modification. Both can be excised with transient piggyBAC transposase expression, leaving no remnant of either modification. Cells with the correct excisions can be selected as follows: (i) 6TG-resistance (the HPRT mini-gene is deleted) and (ii) FIAU-resistance (the puroΔTK gene is deleted). An inversion as described in the Ig loci moves the endogenous IGH-VDJ region away from the Eµ enhancer region, and leads to inactivation of the endogenous IGH-VDJ region.

The resulting transgenic animals have an inactivated endogenous IgH VDJ region and comprise human IgH VDJ gene segments.

### Generation of fully human lgH locus

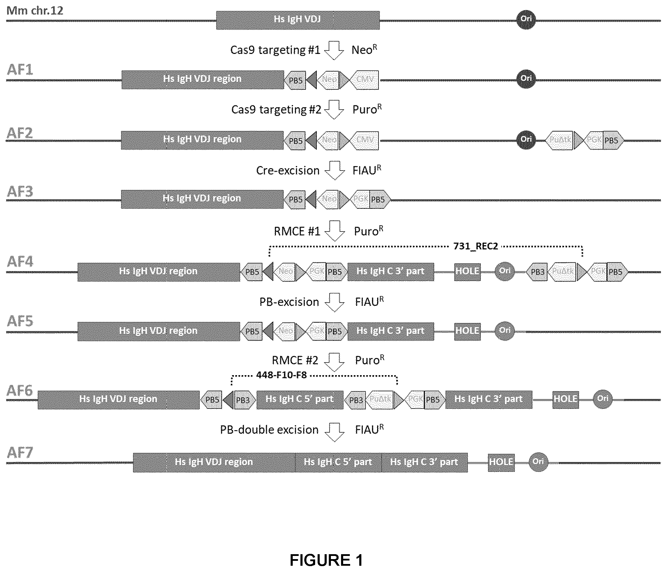

Embryonic stem cells (ESCs) derived from a transgenic mouse as described above, having an inactivated endogenous IgH VDJ region and comprising human IgH V, D and J gene segments, were cultured and modified in order to humanise the constant region of the IgH locus. This involved 7 sequential steps of manipulation, with clonal screening, expansion and QC at each step. The steps are summarised below, and in **Figure 1****:**
1. Insertion of a PiggBac/Lox2272/NeoR/Lox5171/CMV cassette at the 5' end of the IgH constant region i.e. just downstream of the human Js and upstream of the mouse Cµ gene.
2. Insertion of a PuroR/Lox5171/PGK/PiggBac cassette at the 3' end of the IgH constant region i.e. just downstream of the mouse Chr12 origin of replication.
3. Cre recombinase was introduced to ESCs to cause excision of the whole constant region between the two Lox5171 sites.
4. A bacterial artificial chromosome (BAC) containing the 3' 'half' of the human constant region flanked by Lox2272 and Lox5171 sites was inserted by recombination mediated cassette exchange at complementary Lox2272 and Lox5171 sites in the IgH locus.
5. PiggyBac transposase was used to excise residual 3' PiggyBac flanked Puromycin cassette.
6. A BAC containing the 5' 'half' of the human constant region flanked by Lox2272 and LoxP sites was inserted by recombination mediated cassette exchange at complementary Lox2272 and LoxP sites in the IgH locus.
7. PiggyBac transposase was used to excise residual PiggyBac flanked Lox2272 and PiggyBAC flanked Puro/PGK/LoxP.
8. mESCs from step 7 were microinjected into WT C57bl/6 blastocysts and the resulting chimeric founder males were bred to WT C57bl/6 females. F1 animals with transmission of the modified IgH locus were mated to generate a homozygous colony of the strain comprising human IgH VDJC (i.e. fully human IgH).

### Endogenous mouse 'J Deletion'

To ensure complete inactivation of the inverted mouse IgH variable region, the region containing the mouse J gene segments was deleted. This was done using CRISPR Cas9 reagents microinjected into embryos obtained from the strain comprising fully human IgH VDJC (step 8 above).

Two pairs of gRNA sequences flanking the mouse J gene segments were used in combination:

| gRNA ID | Sequence | PAM | gRNA length | Direction |
|---|---|---|---|---|
| HCG013 | GATGCCACTGGGGACGCCAA (SEQ ID NO. 1) | GGG | 20 | For |
| HCG014 | AAGTCCCTTGGCGTCCCCAG (SEQ ID NO. 2) | TGG | 20 | Rev |
| HCG015 | GGAGCCGGCTGAGAGAAGTT (SEQ ID NO. 3) | GGG | 20 | For |
| HCG016 | GTGGAGATAATCTGTCCTAA (SEQ ID NO. 4) | AGG | 20 | Rev |

Microinjected embryos were transferred to pseudo pregnant recipient females and F0 founder animals were screened by PCR for the desired J deletion allele. One allele (HJD5 - deletion of Js but no insertion of the donor sequence) was selected to take forward to generate the "J Deletion" colony.

### Generation of the Kappa knock-out allele

The Kappa KO allele was generated by manipulation of mESCs derived from embryos containing the WT mouse Kappa locus. mESCs were electroporated with a targeting vector containing of a PGK promoter and Neo cassette flanked by 4.5 kb homology arms complementary to the mouse Kappa locus. This facilitated insertion of this cassette around 800bp downstream of the mouse J genes. Insertion at this location results in disrupted rearrangement and/or transcription of the locus giving a Kappa KO phenotype in the mouse strain generated by subsequent microinjection of ESCs, chimera breeding and colony expansion.

### Generation of Lambda knock-out allele

The Lambda KO allele was generated by microinjection of CRISPR Cas9 into embryos carrying the mouse WT Lambda locus. The KO strategy is to create deletion in the endogenous Lambda locus that inactivates Lambda light chain expression.

Inactivation of the Lambda locus can be achieved by deletion or replacement of V2, V3, V1 plus J2/C2 cluster, leaving J3/C3 and J1/1 clusters.

**Figure** 3 illustrates the deletion of 200kb of endogenous mouse Lambda locus to generate Lambda knockout.

Ear clips from F0 founder animals resulting from embryo manipulation were screened by PCR for the deletion and the deletion junction was sequenced. An allele with confirmed incorporation of the donor unique sequence (allele name LKD9) was chosen to take forward for colony generation.

### Example 2: Engineering fully human heavy chain allele containing a deletion of CH1 at Cµ and Cγ1

Embryos from the mice described in Example 1 were further modified using CRISPR-Cas9 to delete CH1 from Cµ and Cγ1. An overview of this two-step deletion strategy is illustrated in **Figure 4****.**

### Generation of Cu CH1 deletion in the lgH locus

The Cµ CH1 deletion was generated by microinjection of CRISPR Cas9 reagents into embryos carrying the humanised IgH locus generated as detailed in Example 1. A pair of gRNAs were designed to direct Cas9 to create double strand breaks flanking the CH1 exon in the humanised Cµ locus. The pair of gRNAs used was as follows:

| gRNA ID | Sequence | PAM | gRNA length | Direction |
|---|---|---|---|---|
| HCG062 | TCACTACTTGCGTCCCGCTG (SEQ ID NO. 5) | TGG | 20 | Rev |
| HCG066 | GCCCAGCCACCGGGACAGAG (SEQ ID NO. 6) | AGG | 20 | For |

F0 founder animals were screened by PCR for deletion between the gRNA sites and/or incorporation of the donor sequence. An allele with a 50bp deletion and a small insertion of an 11 bp 'random sequence' was selected to take forward for colony formation. This allele is referred to herein as `CC1-3' (see **Figure 5**).

### Sequence of CC1-3 allele:

CCTGAGGCCTCACCACGGCCCCGCCACCCCTGATAGCCATGACAGTCTCTCTAGAGAGGGAAGAGAGGGAGC
CGAAGGGGGCGGGAGTGGCGGGCACCGGGCTGACACGTG (SEQ ID NO. 7)

### Generation of a partial genomic CH1 deletion in the humanised Cv1 gene segment

This strategy was designed to create a precise deletion to inactivate expression of Cγ1 CH1 and the CH1 to hinge exon using CRISPR Cas9 and a ssDNA oligo repair template.

These deletion alleles were generated by electroporation of CRISPR Cas9 reagents into embryos homozygous for the human IgH VDJC region sequences with the 'CC1-3' Cµ CH1 deletion allele described above.

A pair of gRNAs were designed to direct Cas9 to create double strand breaks within the Cγ1 CH1 exon and within the CH1 to hinge intron in the humanised Cγ1 locus (**see** **Figure 6**). The pair of gRNAs used was as follows:

| gRNA ID | Sequence | PAM | gRNA length | Direction |
|---|---|---|---|---|
| HCG104 | GATTGGGAGTTACTGGAATC (SEQ ID NO. 8) | TGG | 20 | Rev |
| HCG105 | GCCCCCAGAGGTGCTCTTGG (SEQ ID NO. 9) | AGG | 20 | Rev |

A ssDNA donor template was also included in the reagent electroporation mix, having the following sequence:

CTGCGCCCTGGGCCCAGCTCTGTCCCACACCGCGGTCACATGGCACCACCTCTCTTGCAGAGCCCAAATCTTGT
GACAAAACTCACACATGCCCACCGTGCCCAGGTAAGCCAGCCCAGG (SEQ ID NO. 10)

F0 founder animals were screened by PCR for deletion between the gRNA sites and/or incorporation of the donor sequence. Two alleles with a deletion were selected to take forward for colony formation and investigation. Each allele retains a small region at the 5' of the CH1 with the remainder of the CH1, and a portion of the CH1 to Hinge intron, deleted.

These alleles are referred to herein as Vega2-8 and Vega2-15.

### Sequence of Vega2-8:

CCTCCACCAAGGGCCCATCGGTCTTCCCCCTGGCACCCTCCTCCAATCCAGTAACTCCCAATCT
TCTCTCTGCAG (SEQ ID NO. 11)

### Sequence of Vega2-15:

CCTCCACCAAGGGCCCATCGGTCTTCCCCCTGGCACCCTCCTCCACTGCAGAGAGAAGATTGG
GAGTTACTG (SEQ ID NO. 12)

### Generation of a full CH1 and CH1 to hinge intron deletion in the humanised Cv1 gene segment

These deletion alleles were generated by pronuclear microinjection of CRISPR Cas9 reagents into embryos homozygous for the human IgH VDJC sequences with the 'CC1-3' Cµ CH1 deletion allele.

A pair of gRNAs were designed to direct Cas9 to create double strand breaks 5' upstream of Cγ1 CH1 and within the hinge exon in the humanised Cγ1 locus. The pair of gRNAs used was as follows:

| gRNA ID | Sequence | PAM | gRNA length | Direction |
|---|---|---|---|---|
| HCG069 | TGGGTTCTTAACTGTCCGCG (SEQ ID NO.13 ) | AGG | 20 | Rev |
| HCG108 | CACACATGCCCACCGTGCCC (SEQ ID NO.14) | AGG | 20 | For |

A ssDNA donor template was also included in the reagent electroporation mix. This donor is 234bp in length designed to facilitate a precise deletion of the CH1 and CH1 to hinge intron while replacing the hinge with a modified sequence. The donor is designed to introduce 1 silent mutation 5' of the CH1 and 3 silent mutations in the hinge to prevent further Cas9 activity after repair of the targeted locus. The donor sequence is as follows:

CCAGCCAGGCGCACACCCAATGCCCGTGAGCCCAGACACTGGACGCTGAACGTCGCGGACAGTTAAGAACCC
AcGGGCCTCTGCGCCCTGGGCCCAGCTCTGTCCCACACCGCGGTCACATGGCACCACCTCTCTTGCAGAGCCTA
AATCTTGTGACAAAACTCACACCTGTCCTCCGTGCCCAGGTAAGCCAGCCCAGGCCTCGCCCTCCAGCTCAAG
GCGGGACAGGTGCCC (SEQ ID NO. 15)

F0 founder animals were screened by PCR for deletion between the gRNA sites and/or incorporation of the donor sequence.

**Figure 7** illustrates the generation of alleles with a full deletion of the Cγ1 CH1 and CH1 to hinge intron using a longer ssDNA donor template.

Three alleles, referred to herein as Vega2-28, Vega2-31 and Vega2-32, with different configurations were taken forward.

**Allele Vega2-28:** full deletion and incorporation of donor sequence with modified hinge but with a deletion of 168bp around 5' gRNA site upstream of the hinge (shown in **Figure 8a**).

### Sequence of Vega2-28 allele:

GTCCCTGAGGTGGCACCGATGGCCAGAGCTGAGGCCAAGCTAGAGGCCCTGGACTGTGCTGACTCCCGGCAG
ACACAGAGCGCTGACCTGGCTGCCGAGCCCCGCCCCCGCTGAACGTCGCGGACAGTTAAGAACCCACGGGCC
TCTGCGCCCTGGGCCCAGCTCTGTCCCACACCGCGGTCACATGGCACCACCTCTCTTGCAGAGCCTAAATCTTG
TGACAAAACTCACACCTGTCCTCCGTGCCCAG (SEQ ID NO. 16)

**Allele Vega2-31**: full deletion and incorporation of donor sequence with one base pair mutation within hinge - this is a C to T point mutation that results in a non-deleterious histidine to tyrosine change (H237Y) in the IgG1 coding sequence (shown in **Figure 8b**).

### Sequence of Vega2-31 allele:

AGCCCAGACACTGGACGCTGAACGTCGCGGACAGTTAAGAACCCACGGGCCTCTGCGCCCTGGGCCCAGCTC
TGTCCCACACCGCGGTCACATGGCACCACCTCTCTTGCAGAGCCTAAATCTTGTGACAAAACTTACACCTGTCC
TCCGTGCCCAG (SEQ ID NO. 17)

### Sequence of IgG1 hinge region with point mutation (in bold):

EPKSCDKTYTCPPCP (SEQ ID NO. 18)

**Allele Vega2-32:** full deletion and incorporation of donor sequence with modified hinge but with an insertion of 24bp (a duplication of the adjacent sequence in the 5' homology arm of the repair donor) around 5' gRNA site upstream of the hinge (shown in **Figure 8c**).

### Sequence of Vega2-32 allele:

CCAGCCAGGCGCACACCCAATGCCCGTGAGCCCAGACACTGGACGCTGAACCTCGCCCAGACACTGGACGCT
GAACGTCGCGGACAGTTAAGAACCCAcGGGCCTCTGCGCCCTGGGCCCAGCTCTGTCCCACACCGCGGTCACA
TGGCACCACCTCTCTTGCAGAGCCTAAATCTTGTGACAAAACTCACACCTGTCCTCCGTGCCCAG (SEQ ID NO.
19)

### Example 3: B cell development

### Normal 6 Cell Compartments in Transgenic Mice described herein

In mice, about 2 × 10⁷ bone marrow immature B cells are produced daily. Among them, only 10-20% of these cells survive to exit the bone marrow and enter the spleen. The immature splenic B cell population is divided into two distinct subsets: transitional 1 (T1) and transitional 2 (T2) B cells. In vivo experiments indicate that T1 cells give rise to T2 cells, whereas T2 cells can further differentiate into mature (M) B cells. In contrast to immature B cells (3-4 days old), mature B cells are long-lived (15-20 weeks old) and are ready to respond to antigens (Pillai S et a I; Immunol. Reviews. 2004. 197: 206-218). Thus, the component of mature B cell population is directly linked to the efficiency of humoral immune response.

The T1, T2 and M cell populations can be categorized by their cell surface IgM and IgD levels. A normal phenotype of splenic B cell compartment is required to mount a robust immune response.

### Methods

Flow cytometric analysis of mature B lymphocytes: To obtain a single cell suspension from spleen, the spleens of mice listed below were gently passaged through a 30µm cell strainer. Single cells were resuspended in PBS supplemented with 3% heat inactivated foetal calf serum (FCS; Gibco^{®}). The following antibodies were used for staining:
- Antibody against B220/CD45R conjugated with allophycocyanin (APC) (eBioscience, clone RA3-6B2),
- antibody against IgD receptor conjugated with phycoerythrin (PE) (eBioscience, clone 1 1 -26) and
- IgM receptor conjugated with fluorescein isothiocyanate (FITC) (eBioscience, clone 1 1/41).

5×10⁶ cells were used for each staining. To each vial containing splenocytes a cocktail of antibodies was added consisting of: IgD (PE) (eBioscience, clone 1 1-26), IgM (FITC) and B220/CD45R (APC). Cells were incubated at 6°C for 15 minutes, washed to remove excess of unbound antibodies and analysed using a fluorescence-activated cell sorting (FACS) analyser from Miltenyi Biotech. B-cells were gated as B220 + IgM + IgD" for T1 population, B220 + IgM + IgD + for T2 population and B220 + IgM + IgD + for M population. Percentage of cells was calculated using gating system.

### Results

Spleens from the mice described herein were collected and analysed for their B cell compartments. The number and percentages of T1, T2 and M cells among those mice are similar compared to wildtype, indicating that genetic manipulation of endogenous IG loci in transgenic mice according to the invention do not compromise their B cell development. These data help to establish that animals according to the invention provide a robust platform for antibody discovery.

As explained, normal splenic and bone marrow compartments are seen in such mice of the invention (i.e., equivalent to the compartments of mice expressing only mouse antibody chains).

### Example 4: Methods for analysis of class-switching, affinity maturation and response to antigen challenge in transgenic mice described herein

Transgenic animals known in the art for producing human HCAbs (e.g. platforms developed by Harbour Biomed, Crescendo Biologics and Teneobio Inc. (UNIRAT) are not able to undergo class switching because the entire Cµ region is deleted or the CH1 region of every constant gene is deleted.

In contrast, the transgenic non-human animal cells described herein provide a platform for producing human HCAbs are able to undergo allows class-switching from IgM to IgG1. In particular, the CH1 deletions described herein lead to correct splicing of VH to CH2 for IgM and to the hinge region for IgG.

*Mice that have not been immunised:* Spleens and lymph nodes were collected from naïve mice and processed for sorting. For this particular example, these mice were older than used as standard, ~14 weeks of age.

*Immunised mice:* transgenic mice at 6-8 weeks of age were primed by subcutaneous injection with 20ug target protein suspended in phosphate buffer saline (PBS) and equivalent volume of Addavax as adjuvant. The immunized mice were boosted three times with 10ug of target protein in PBS and same volume of Addavax as adjuvant every three weeks after priming. Final boost was administrated three weeks after the last boost using 5ug of target protein in PBS without adjuvant. Five days after the final boost spleens and lymph nodes were collected and processed for sorting.

### Spleen and lymph node cell isolation, and 6 cell enrichment

Splenocytes were isolated from spleens using the flushing method. Briefly, a full spleen was flushed with approximately 10 ml of 10% FBS/RPMI w/o phenol red buffer or until the spleen has lost its red colour. The cell suspension was periodically pipetted onto 40µm cell strainer. Homogenise LN cells were filtered in the same 40µm cell strainers used for the spleens. Cells were pelleted at 400xg for 10 min at RT and resuspend in 5mL 3%FBS/1mM EDTA/RPMI before proceeding with B cell enrichment by magnetic beads technologies.

### lgG+ 6 cell sorting from Naïve mice

B cell enriched samples were resuspended in 300µl of 3%FBS/RPMI w/o phenol red. 20µl of Fc blocker was added and incubated for 10 min on ice.100µl of staining cocktail containing B220-BUV395, CD19-BUV395, CD4-BV510, CD8α-BV510, Ly6G-BV510, F4/80-BV510, IgM-BV650, IgG-PE. Samples were incubated for 30min on ice. Cells were pelleted at 400xg for 10min at 4°C and resuspended to 800µl before sorting. IgG+ B cells were collected and used to retrieve VDJ sequences.

### Antigen specific 8 cell sorting

B cell enriched samples were resuspended in 300µl of 3%FBS/RPMI w/o phenol red. 20µl of Fc blocker was added and incubated for 10 min on ice.100µl of staining cocktail containing B220-BUV395, CD19-BUV395, CD4-BV510, CD8α-BV510, Ly6G-BV510, F4/80-BV510, IgM-BV650, IgG-PE, CD138-BV711, and target proteins conjugated with -PE or -APC were added to each sample. Samples were incubated for 30min on ice. Cells were pelleted at 400xg for 10min at 4°C and resuspended to 800µl before sorting. Live cells were gated to exclude doublets, and non-B cells using CD4/CD8/Ly6G/F4-80 markers. CD138+, IgM- cells were sorted as a plasmablast/plasma cell population. CD138- CD19/B220+, IgM-, IgG+, Ag+ cells were sorted as an antigen positive B cell population. Both antigen positive B cells and plasmablast/plasma cells collected were used to retrieve VDJ sequences.

### VDJ Sequence Recovery and analysis using microfluidics

Sorted cells were processed using standard kits manufactured by 10x Genomics: Chromium Single Cell 5' Reagent Kits (v2 Chemistry Dual Index); and the Chromium Single Cell Human BCR amplification Kit as per manufacturer's protocol. Additional IgM & IgG1 CH2 specific oligos were spiked in at the VDJ amplification stage to capture the CH1 deleted transcripts.

Briefly, after the sort, cells were loaded on a Chromium Next GEM chip K to form GEMs (Gel bead-in-EMulsion) using 10x Genomics Controller. GEMs were transferred to a PCR tube and incubated on a thermocycle for the RT reaction to generate cDNA. cDNA was purified with magnetic beads and amplified as described by the 10x Genomics protocol. After amplification the samples were cleaned using SPRIselect reagents. The resulting amplified cDNA was VDJ enriched by two sequential PCR using the Mouse BCR amplification kit spiking in at each round primers targeting the constant region upward of the deleted CH1 . The enriched material was then process as per protocol to construct an NGS library, then sequenced on a NextSeq2000. Sequencing data were analysed using 10x Genomics Cell Ranger 3.0.0.

### Class-switching and affinity maturation in non-immunized animals

In order to provide early evidence for this feature, spleens and lymph nodes from non-immunized animals homozygous for one of two heavy locus alleles (Vega2-8 and Vega2-15), plus the lambda and kappa knockout alleles, were harvested and analysed using the 10X platform. B cells were sorted using a panel of markers and scRNA-seq experiments and NGS (Next Generation Sequence) analyses were performed to further demonstrate that the transgenic mice can class switch from IgM to IgG1.

### Results from non-immunized animals

The rearranged transcripts can be detected Chromium Single Cell 5' Reagent Kits (v2 Chemistry Dual Index) with Immune Receptor Mapping spiked with hinge and CH2 specific primers from spleen and lymph nodes cells of naive and immunized transgenic mice. Further sequence analysis of these amplified fragments demonstrated hypermutation happened within the human variable region of these IgG chains.

These results indicate that the transgenic locus described herein comprising insertion of human heavy chain sequence, and deletion of CH1 in Cµ and Cγ1 allows class switching from IgM to IgG.

The data is summarised in Table 1 below.

**Table 1**

| Criteria | Vega2-8 mouse #1 | Vega2-8 mouse #2 | Vega2-15 mouse #1 | Vega2-15 mouse #2 |
|---|---|---|---|---|
| Total cells after B cell enrichment | 8.85x10^6 | 6.36x10^6 | 10.6x10^6 | 8.34x10^6 |
| Total cells sorted and analysed | 8371 | 15078 | 24001 | 18539 |
| Total sequences recovered | 761 | 2454 | 3381 | 2499 |
| Total high quality IgM sequences | 583 | 2196 | 2856 | 2161 |
| Total high quality IgG sequences | 111 | 70 | 215 | 126 |
| Highest nucleotide mutations | 7 | 8 | 9 | 8 |

Despite the non-immunised state of these animals, a significant number of heavy chain-only IgG-presenting B cells were isolated, confirming class-switching from heavy-chain only IgM to IgG as illustrated in Table 1.

Sequence analysis of the transcripts produced by the B cells isolated also showed that there is mutation away from germline sequences and some clusters of sequences i.e. related sequences derived from a common VDJ recombination event but with different levels of somatic hypermutation, suggested active clonal expansion.

### EMBODIMENTS OF THE DISCLOSURE

1. A non-human animal cell comprising an antibody heavy chain locus comprising a plurality of human IgH V gene segments, one or more human IgH D gene segments and one or more human IgH J gene segments upstream of all or part of a human heavy constant (C) region and forming a human heavy VDJC region, wherein:
   i) the human heavy VDJC DNA is inserted upstream, downstream or at the endogenous non-human animal IgH locus;
   ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated; and
   iii) there is substantially no expression of light chains and substantially no expression of non-human heavy chains.
2. The cell of embodiment 1, wherein said cell produces antibodies of both IgG and IgM isotypes and is capable of undergoing isotype switching.
3. The cell of any preceding embodiment, wherein the Cµ CH1 domain and/or the Cγ1 CH1 domain is inactivated by partial deletion of the nucleotide sequence encoding the CH1 domain.
4. The cell of any preceding embodiment, wherein the Cµ CH1 domain and/or the Cγ1 CH1 domain is inactivated by full deletion of the nucleotide sequence encoding the CH1 domain.
5. The cell of any preceding embodiment, wherein the Cµ CH1 domain is inactivated by full deletion of the nucleotide sequence encoding the CH1 domain and the Cγ1 CH1 domain is inactivated by partial deletion of the nucleotide sequence encoding the CH1 domain.
6. The cell of embodiment 4, wherein the Cµ CH1 domain is inactivated by full deletion of the nucleotide sequence encoding the CH1 domain and the Cγ1 CH1 domain is inactivated by full deletion of the nucleotide sequence encoding the CH1 domain.
7. The cell of any preceding embodiment, wherein the endogenous non-human animal heavy chain VDJ gene segments are inverted.
8. The cell of any preceding embodiment, wherein one or more of the endogenous heavy chain J gene segments have been deleted.
9. The cell of any preceding embodiment, wherein the inserted human heavy chain VDJ region comprises, in germline configuration, all of the V, D and J gene segments and intervening sequences from a human.
10. The cell of any preceding embodiment, wherein the inserted human heavy chain DNA includes, preferably consists of, the complete human heavy chain VDJC region.
11. The cell of any preceding embodiment, wherein the non-human animal is a rodent, preferably a mouse or a rat.
12. The cell of any preceding embodiment, wherein the endogenous lambda locus retains JC1 and/or JC3 gene segments.
13. The cell of any preceding embodiment, wherein all endogenous lambda and/or kappa V genes are deleted.
14. The cell of any preceding embodiment, wherein there is no expression of light chains and no expression of non-human heavy chains.
15. The cell of any preceding embodiment, wherein the heavy chain constant region comprises Cδ, Cε, Cγ2, Cγ3 and Cα gene segments each encoding a CH1 domain.
16. The cell of any preceding embodiment, wherein the Cµ gene segment is located upstream of the Cγ1 gene segment.
17. The cell of any preceding embodiment, wherein the Cγ1 gene segment comprises a point mutation resulting in a H237Y mutation in the IgG1 coding sequence.
18. The cell of any preceding embodiment, wherein the antibody heavy chain locus comprises a fully human constant region.
19. The cell of any preceding embodiment, wherein the cell is homozygous for said antibody heavy chain locus.
20. A non-human animal comprising a plurality of cells according to any preceding embodiment, wherein the animal is capable of expressing a repertoire of IgM and IgG heavy chain only antibodies.
21. A method for producing a human heavy chain only antibody specific to a desired antigen, the method comprising immunising a non-human animal according to embodiment 20 with the desired antigen and recovering the antibody or its encoding nucleic acid or recovering a cell producing the antibody from the animal.
22. A method according to embodiment 21, further comprising cloning the encoding nucleic acid into a recombinant host cell, culturing the cell for expression of the heavy chain antibody, and recovering and purifying the antibody from the cell or culture medium.
23. The method of embodiment 21 or embodiment 22, further comprising formulating the antibody or antibody chain with a pharmaceutically acceptable carrier or other excipient to produce a pharmaceutical composition.
24. A method of producing a pharmaceutical composition comprising an antibody specific to a desired antigen and a pharmaceutically acceptable carrier or other excipient, the method comprising immunising a non-human animal according to embodiment 20, with the desired antigen and recovering the antibody and formulating the antibody with the pharmaceutically acceptable carrier or other excipient.

## Claims

1. A non-human animal cell comprising an antibody heavy chain locus comprising a plurality of human IgH V gene segments, one or more human IgH D gene segments and one or more human IgH J gene segments upstream of all or part of a human heavy constant (C) region and forming a human heavy VDJC region, wherein:
i) the human heavy VDJC DNA is inserted upstream, downstream or at the endogenous non-human animal IgH locus;
ii) the human heavy chain constant region comprises Cδ, Cε, Cγ2, Cγ3 and/or Cα gene segments each encoding CH1 domains and comprises Cµ and Cγ1 gene segments, wherein the Cµ CH1 domain and the Cγ1 CH1 domain are inactivated; and
iii) there is substantially no expression of light chains and substantially no expression of non-human heavy chains.

2. The cell of claim 1, wherein said cell produces antibodies of both IgG and IgM isotypes and is capable of undergoing isotype switching.

3. The cell of any preceding claim, wherein the Cµ CH1 domain and/or the Cγ1 CH1 domain is inactivated by partial deletion of the nucleotide sequence encoding the CH1 domain.

4. The cell of any preceding claim, wherein the Cµ CH1 domain and/or the Cγ1 CH1 domain is inactivated by full deletion of the nucleotide sequence encoding the CH1 domain.

5. The cell of any preceding claim, wherein the Cµ CH1 domain is inactivated by full deletion of the nucleotide sequence encoding the CH1 domain and the Cγ1 CH1 domain is inactivated by partial deletion of the nucleotide sequence encoding the CH1 domain.

6. The cell of claim 4, wherein the Cµ CH1 domain is inactivated by full deletion of the nucleotide sequence encoding the CH1 domain and the Cγ1 CH1 domain is inactivated by full deletion of the nucleotide sequence encoding the CH1 domain.

7. The cell of any preceding claim, wherein one or more of the endogenous heavy chain J gene segments have been deleted.

8. The cell of any preceding claim, wherein the inserted human heavy chain VDJ region comprises, in germline configuration, all of the V, D and J gene segments and intervening sequences from a human.

9. The cell of any preceding claim, wherein the non-human animal is a rodent, preferably a mouse or a rat.

10. The cell of any preceding claim, wherein the endogenous lambda locus retains JC1 and/or JC3 gene segments.

11. The cell of any preceding claim, wherein all endogenous lambda and/or kappa V genes are deleted, preferably wherein there is no expression of light chains and no expression of non-human heavy chains.

12. The cell of any preceding claim, wherein the Cµ gene segment is located upstream of the Cγ1 gene segment.

13. The cell of any preceding claim, wherein the Cγ1 gene segment comprises a point mutation resulting in a H237Y mutation in the IgG1 coding sequence.

14. A non-human animal comprising a plurality of cells according to any preceding claim, wherein the animal is capable of expressing a repertoire of IgM and IgG heavy chain only antibodies.

15. A method for producing a human heavy chain only antibody specific to a desired antigen, the method comprising immunising a non-human animal according to claim 14 with the desired antigen and recovering the antibody or its encoding nucleic acid or recovering a cell producing the antibody from the animal.

16. A method according to claim 15, further comprising cloning the encoding nucleic acid into a recombinant host cell, culturing the cell for expression of the heavy chain antibody, and recovering and purifying the antibody from the cell or culture medium.

17. A method of producing a pharmaceutical composition comprising an antibody specific to a desired antigen and a pharmaceutically acceptable carrier or other excipient, the method comprising immunising a non-human animal according to claim 14, with the desired antigen and recovering the antibody and formulating the antibody with the pharmaceutically acceptable carrier or other excipient.
